# EUROPEAN PATENT SPECIFICATION

(11) **EP 3 320 908 B1**
(45) Date of publication and mention of the grant of the patent: **06.09.2023**
(21) Application number: 16821404.7
(22) Date of filing: 05.07.2016
(51) Int. Cl.: A61K 35/74, A61K 9/20, A61K 35/742, A61K 35/744, A61K 47/04, A61K 47/14, A61K 47/26, A61K 47/32, A61K 47/36, A61K 47/38, A61P 1/04, A61P 1/10, A61P 1/12, A61P 1/14, A61P 31/04, A61P 37/04, A61K 9/00, A61P 43/00

(54) **BACTERIA-CONTAINING ORAL RAPIDLY DISINTEGRATING TABLET**
BAKTERIENHALTIGE, ORAL SCHNELL ZERFALLENDE TABLETTE
COMPRIMÉ À DÉSINTÉGRATION ORALE RAPIDE CONTENANT DES BACTÉRIES

(30) Priority: 07.07.2015 JP 2015136163
(43) Date of publication of application: 16.05.2018
(73) Proprietor: Toa Pharmaceutical Co., Ltd., Tokyo 151-0073 (JP)
(72) Inventor: KOSHIISHI,Daisuke, Tokyo 151-0073 (JP); TANAKA,Tadao, Tokyo 151-0073 (JP)
(74) Representative: Hertin und Partner Rechts- und Patentanwälte PartG mbB
(86) International application number: PCT/JP2016/069920
(87) International publication number: WO 2017/006935

(56) References cited:
- EP-A1- 1 281 752
- WO-A1-2007/111375
- WO-A1-2007/111375
- JP-A- H0 441 434
- JP-A- 2002 017 337
- JP-A- 2014 133 736
- US-A1- 2012 014 923
- KLAYRAUNG S ET AL: "Development of tablets containing probiotics: Effects of formulation and processing parameters on bacterial viability", INTERNATIONAL JOURNAL OF PHARMACEUTICS, ELSEVIER, NL, vol. 370, no. 1-2, 31 March 2009 (2009-03-31), pages 54-60, XP026001807, ISSN: 0378-5173, DOI: 10.1016/J.IJPHARM.2008.11.004 [retrieved on 2008-11-18]
- KEI OGUCHI ET AL.: 'Ryudoso Zoryu to Kakuhan Zoryu ni yoru Seimitsu na Zoryu Hoho no Kento' KATEIYAKU KENKYU vol. 31, March 2012, pages 70 - 5, XP009508194

## Description

### Technical Field

The present invention relates to a bacterium-containing oral rapidly disintegrating tablet, and more particularly, to a viable bacterium-containing oral rapidly disintegrating tablet. More specifically, the present invention relates to a bacterium-containing oral rapidly disintegrating tablet excellent in long-term stability of viable bacteria.

The present application claims priority from Japanese Patent Application No. 2015-136163.

### Background Art

### (Oral Rapidly Disintegrating Tablet)

An oral rapidly disintegrating tablet has a feature of dissolving or disintegrating with saliva or a small amount of water in the mouth, and is easy to take even for a patient or elderly person who has a low ability to swallow. In addition, it is generally desired that a period of time required for the dissolution or disintegration in the mouth be as short as possible.

### (Probiotic)

Probiotics have a wide range of action mechanisms. Basically, however, the following actions are conceivable: viable bacteria that have reached the inside of the intestines inhibit directly or indirectly contact of pathogens with the intestinal epithelium (competitive exclusion); pathogens are excluded by an organic acid or hydrogen peroxide produced in the intestinal tract by viable bacteria that have reached the inside of the intestines (chemical inhibition); and viable bacteria that have reached the inside of the intestines stimulate the immune system of a host, to thereby enhance the biological defense function of the host (immunomodulation). There are reports of pathogen growth suppression, development promotion, improvement in feed conversion ratio, improvement in laying performance, and the like caused by administration of probiotics.

The applicant of the present application sells probiotics containing lactic acid bacteria *Streptococcus faecalis* T-110 (also called *"Enterococcus faecium* T-110"), butyric acid bacteria *Clostridium butyricum* TO-A, and saccharifying bacteria *Bacillus mesentericus* TO-A (also called *"Bacillus subtilis* TO-A". However, those probiotics each have a shape not of an oral rapidly disintegrating tablet, but of a powder or a plain tablet (uncoated tablet).

### (Related Patent Literatures)

Related art concerning oral rapidly disintegrating tablets containing bacteria is as described below.

In Patent Literature 1, there is disclosed an "oral rapidly disintegrating tablet containing useful bacteria, microcrystalline cellulose/light anhydrous silicic acid, and a starch-containing saccharide, and having a moisture content of 3.2 wt% or less, the tablet being obtained by compression-molding mixture powder of the useful bacteria, the microcrystalline cellulose/light anhydrous silicic acid, and the starch-containing saccharide in a dry state, in which the tablet contains, with respect to 100 parts by weight of the tablet, 35 parts by weight to 70 parts by weight of the microcrystalline cellulose/light anhydrous silicic acid, and 25 parts by weight to 60 parts by weight of the starch-containing saccharide, and has a tablet hardness of from 30 N to 60 N."

In Patent Literature 2, there is disclosed a "production method for an orally disintegrating tablet containing lactic acid bacteria or an extracted component thereof, the tablet keeping oral easy disintegrability and tablet hardness, the method being characterized by: mixing a) a granulated product containing, as an active component, lactic acid bacteria or an extracted component thereof, and being granulated with an excipient and/or a moisture-controlling agent being blended, and b) powder for an orally disintegrating tablet having easy disintegrability, the powder containing a sugar and/or a sugar alcohol, and not containing lactic acid bacteria or an extracted component thereof; and molding the mixture."

In Patent Literature 3, tablets are disclosed having gastric acid tolerance by providing enteric coating on the tablets produced by mixing a viable cell powder of Lactobacillus and additive(s) such as starch.

In Patent Literature 4, a method for the manufacture of a dried microorganism cell product using spray drying is disclosed.

In Patent Literature 5, a hypoglycemic effect enhancer is disclosed for enhancing the activity of an α-glucosidase inhibitor, the enhancer comprising at least one kind of bacteria selected from the group consisting of bifidobacteria, lactic acid bacteria, saccharifying bacteria, and butyric acid bacteria.

A production method for a bacterium-containing oral rapidly disintegrating tablet of the present invention includes a step of granulating bacteria by a spray-drying method unlike the production method for the bacterium-containing oral rapid disintegration-type tablet described in any one of the above-mentioned patent literatures.

### Citation List

### Patent Literature

[PTL 1] JP 5100634 B2
[PTL 2] JP 2012-41293 A
[PTL 3] JP H04 41434 A
[PTL 4] EP 1281752 A1
[PTL 5] US 2012/014923 A1

### Summary of Invention

### Technical Problem

An oral rapidly disintegrating tablet containing a probiotic has not been put on the market yet, and is difficult to formulate. In view of this, an object of the present invention is to provide, by a novel tablet production method, a bacterium-containing oral rapidly disintegrating tablet, in particular, a viable bacterium-containing oral rapidly disintegrating tablet, more specifically a bacterium-containing oral rapidly disintegrating tablet excellent in long-term stability of viable bacteria.

### Solution to Problem

The inventors of the present invention have made extensive investigations in order to achieve the above-mentioned object. The inventors have found that a disintegration time can be shortened and stability of viable bacteria can be improved by using a spray-drying method in a process of granulating viable bacteria. Thus, the inventors have completed the present invention.

The invention therefore relates to the subject matter of claim 1 as appended below, namely to a bacterium-containing oral rapidly disintegrating tablet, comprising:
bacteria comprising viable bacteria;
a saccharide;
an excipient;
a disintegrant; and
a lubricant,
the bacterium-containing oral rapidly disintegrating tablet being obtained by compression-forming mixture powder containing the bacteria comprising viable bacteria granulated by a spray-drying method, the saccharide, the excipient, the disintegrant, and the lubricant,
the bacterium-containing oral rapidly disintegrating tablet having a tablet hardness of from 15 N to 50 N.

The invention further relates to the subject matter of claim 8 as appended below, namely to a production method for a bacterium-containing oral rapidly disintegrating tablet, comprising the steps of:
(1) granulating bacteria that comprise viable bacteria by a spray-drying method;
(2) mixing the bacteria granulated in the step (1), a saccharide, an excipient, a disintegrant, and a lubricant to produce mixture powder; and
(3) compression-forming the mixture powder of the step (2) so as to achieve a tablet hardness of from 15 N to 50 N.

Further embodiments are presented in the description and dependent claims as below.

### Advantageous Effects of Invention

The bacterium-containing oral rapidly disintegrating tablet of the present invention has excellent oral rapid disintegrability and excellent stability of viable bacteria.

### Brief Description of Drawings

FIGS. 1 are photographs for showing results of observation of the shapes of bacterial powders.
FIG. 2 is a graph for showing the particle size distributions of particles of bacterial powders of lactic acid bacteria.
FIG. 3 is a schematic diagram of measurement positions for an angle of repose.
FIGS. 4 are graphs for showing investigation results of the stability of bacterial powders {acceleration conditions (under a 40°C-75%RH (relative humidity) environment)}.
FIGS. 5 are graphs for showing investigation results of the stability of bacterium-containing oral rapidly disintegrating tablets {acceleration conditions (under a 40°C-75%RH environment)}.

### Description of Embodiments

The present invention relates to a bacterium-containing oral rapidly disintegrating tablet, and more particularly, to a viable bacterium-containing oral rapidly disintegrating tablet. More specifically, the present invention relates to a bacterium-containing oral rapidly disintegrating tablet excellent in long-term stability of viable bacteria. Now, the present invention is described in detail.

### (Bacterium-containing Oral Rapidly Disintegrating Tablet of the Present Invention)

A bacterium-containing oral rapidly disintegrating tablet of the present invention contains bacteria, a saccharide, an excipient, a disintegrant, and a lubricant, the bacterium-containing oral rapidly disintegrating tablet being obtained by compression-forming mixture powder containing the bacteria (in particular, viable bacteria) granulated by a spray-drying method, the saccharide, the excipient, the disintegrant, and the lubricant.

### (Bacteria)

The bacteria contained in the bacterium-containing oral rapidly disintegrating tablet of the present invention are not particularly limited as long as the bacteria are bacteria granulated by the spray-drying method. Examples thereof include, but not particularly limited to, lactic acid bacteria, butyric acid bacteria, hay bacilli, saccharifying bacteria, and bifidobacteria.

The bacteria contained in the bacterium-containing oral rapidly disintegrating tablet of the present invention include, for example, at least bacteria of one or more or two or more members selected from lactic acid bacteria, butyric acid bacteria, and hay bacilli.

The hay bacilli are preferably saccharifying bacteria. In addition, the lactic acid bacteria are preferably *Streptococcus faecalis,* more preferably *Streptococcus faecalis* T-110 (also called *"Enterococcus faecium* T-110"). The butyric acid bacteria are preferably *Clostridium butyricum,* more preferably *Clostridium butyricum* TO-A. The saccharifying bacteria are preferably *Bacillus mesentericus,* more preferably *Bacillus mesentericus* TO-A (also called *"Bacillus subtilis* TO-A").

The above-mentioned bacteria are viable bacteria.

The above-mentioned bacteria are available from Toa Pharmaceutical Co., Ltd.

The blending amount of the bacteria (viable bacterial cells, wet bacterial cells, or dry bacterial cells) contained in the bacterium-containing oral rapidly disintegrating tablet of the present invention is not particularly limited, but is from 0.1 part by weight to 50 parts by weight, preferably from 1.0 part by weight to 40 parts by weight, more preferably from 10 parts by weight to 35 parts by weight, still more preferably from 15 parts by weight to 27 parts by weight, with respect to 100 parts by weight of the tablet.

In addition, the number of the bacteria contained in the bacterium-containing oral rapidly disintegrating tablet of the present invention is from 10³ to 10²⁰, preferably from 10⁵ to 10¹⁵, more preferably from 10⁷ to 10¹³, per tablet.

### (Saccharide)

The saccharide contained in the bacterium-containing oral rapidly disintegrating tablet of the present invention is not particularly limited as long as the saccharide is a saccharide to be used in a general tablet, and examples thereof may include lactose (lactose hydrate), glucose, fructose, lactulose, saccharose, maltose, powder candy, maltose, trehalose, and sucrose. In addition, the saccharide may also be used as an excipient.

The blending amount of the saccharide contained in the bacterium-containing oral rapidly disintegrating tablet of the present invention is not particularly limited, but is from 10 parts by weight to 90 parts by weight, preferably from 30 parts by weight to 85 parts by weight, more preferably from 50 parts by weight to 80 parts by weight, still more preferably from 70 parts by weight to 75 parts by weight, with respect to 100 parts by weight of the tablet.

### (Excipient)

The excipient contained in the bacterium-containing oral rapidly disintegrating tablet of the present invention is not particularly limited as long as the excipient is an excipient to be used in a general tablet, and examples thereof may include, in addition to the above-mentioned saccharides, anhydrous dibasic calcium phosphate, starch (potato starch, corn starch, or wheat starch), cellulose (microcrystalline cellulose), hydroxypropylcellulose, maltitol, mannitol, sorbitol, xylitol, and erythritol.

The blending amount of the excipient (including the saccharide) contained in the bacterium-containing oral rapidly disintegrating tablet of the present invention is not particularly limited, but is from 10 parts by weight to 90 parts by weight, preferably from 30 parts by weight to 85 parts by weight, more preferably from 50 parts by weight to 80 parts by weight, still more preferably from 70 parts by weight to 75 parts by weight, with respect to 100 parts by weight of the tablet.

### (Disintegrant)

The disintegrant contained in the bacterium-containing oral rapidly disintegrating tablet of the present invention is not particularly limited as long as the disintegrant is a disintegrant to be used in a general tablet, and examples thereof may include crospovidone, light anhydrous silicic acid, low-substituted hydroxypropylcellulose, carmellose calcium, croscarmellose sodium, and sodium carboxymethyl starch.

### (Lubricant)

The lubricant contained in the bacterium-containing oral rapidly disintegrating tablet of the present invention is not particularly limited as long as the lubricant is a lubricant to be used in a general tablet, and examples thereof may include talc, sodium stearyl fumarate, magnesium stearate, a sucrose fatty acid ester, calcium stearate, a hydrogenated oil, polyethylene glycol, and sodium lauryl sulfate.

### (Other Component)

The bacterium-containing oral rapidly disintegrating tablet of the present invention may optionally contain a binder, an acidulant, a foaming agent, an artificial sweetener, a colorant, a stabilizing agent, or the like known per se.

### (Tablet Hardness)

The tablet hardness of the bacterium-containing oral rapidly disintegrating tablet of the present invention is from 15 N to 50 N, preferably from 20 N to 40 N.

### (Moisture Content)

The moisture content of the bacterium-containing oral rapidly disintegrating tablet of the present invention is 3% or less, preferably 2% or less.

### (Disintegration Time of Tablet)

An oral disintegration time that is the disintegration time of the bacterium-containing oral rapidly disintegrating tablet of the present invention is from 10 seconds to 30 seconds, preferably from 10 seconds to 25 seconds, more preferably from 10 seconds to 23 seconds, still more preferably from 10 seconds to 20 seconds, most preferably from 10 seconds to 19 seconds, in the mouth.

### (Stability of Viable Bacteria in Tablet)

The stability of viable bacteria in the bacterium-containing oral rapidly disintegrating tablet of the present invention is as follows: when the amount of the viable bacteria on the day of the production of the tablet is defined as 100, the amount of the viable bacteria is from about 95 to about 100 after a lapse of 3 months, and an amount of the viable bacteria of from about 90 to about 98 (or from about 90 to about 100) can be maintained after a lapse of 6 months.

### (Shape and Mass of Tablet)

The shape of the bacterium-containing oral rapidly disintegrating tablet of the present invention is not particularly limited, but is desirably a round shape, a triangular shape, a ball shape, or the like. In addition, the mass of the tablet is not particularly limited, but falls within the range of, for example, from 85 mg to 115 mg.

### (Production Method for Bacterium-containing Oral Rapidly Disintegrating Tablet)

The main steps of a production method for the bacterium-containing oral rapidly disintegrating tablet of the present invention include: (1) a step of culturing viable bacteria; (2) a step of granulating bacteria (viable bacteria) by a spray-drying method; (3) a step of producing mixture powder; and (4) a compression-forming step.

The most different feature of the production method for the bacterium-containing oral rapidly disintegrating tablet of the present invention as compared to a related-art bacterium-containing oral rapidly disintegrating tablet is the "step of granulating bacteria (viable bacteria) by a spray-drying method" instead of granulating bacteria (viable bacteria) by a freeze-drying method.

In the step of granulating bacteria (viable bacteria) by the freeze-drying method, moisture-containing wet bacterial cells (viable bacterial cells) are frozen, and then dried under reduced pressure into a cake-like mass. Accordingly, a step of pulverizing the mass and a sizing step for regulating particle diameters are required. As a result, base powder obtained by the freeze-drying method is formed of amorphous particles and also has a large particle size distribution width.

### (Step of Culturing Viable Bacteria)

Bacterial seeds are placed in a culture apparatus known per se, and bacterial cells are cultured by a method known per se. Next, after the completion of the culture, centrifugation is performed to provide wet bacterial cells (viable bacterial cells).

### (Step of Granulating Bacteria (Viable Bacteria) by Spray-drying Method)

The wet bacterial cells (viable bacterial cells) obtained in the foregoing are sprayed into a spray dryer chamber to become a mist, and are dried with hot air to form fine particles. Because of a surface tension, the viable bacterial cells mainly become spherical particles. For example, the following apparatus, conditions, and the like may be utilized.
Granulation apparatus: Spray Dryer Model ODA-20 (Ohkawara Kakohki Co., Ltd.)
Conditions at time of granulation: inlet: 110°C to 130°C, outlet: 70°C to 90°C, number of rotations: 2,000 rpm to 15,000 rpm, liquid feed amount: 28.1 HZ (150 ml/min to 200 ml/min)
Other item to note: The liquid feed amount is adjusted as necessary.

### (Method of Producing Mixture Powder)

The bacteria granulated in the above-mentioned step, the saccharide, the excipient, the disintegrant, and the lubricant are weighed out, and mixed using a mixer known per se {rotary rocking mixer (2500 L)} for from several minutes to several hours to provide mixture powder. The mixture powder is stored in a polyethylene container until the next step.

### (Compression-forming Step)

The mixture powder obtained in the above-mentioned step is compression-formed using a tableting machine known per se at a tableting pressure set to the range of from 5 kN to 10 kN, to thereby provide a bacterium-containing oral rapidly disintegrating tablet having a tablet hardness in the range of from 20 N to 30 N.

### (Storing Step)

A storing method for the bacterium-containing oral rapidly disintegrating tablet of the present invention obtained in the above-mentioned step is not particularly limited as long as the storing method is a storing method for a tablet known per se, but the tablet is stored in a bottle containing a desiccant.

The bacterium-containing oral rapidly disintegrating tablet of the present invention has a combination of any one, two, three, four, five, six, or all of the following features (1) to (7).
(1) The blending amount of the bacteria contained in the bacterium-containing oral rapidly disintegrating tablet is from 0.1 part by weight to 50 parts by weight, preferably from 1.0 part by weight to 40 parts by weight, more preferably from 10 parts by weight to 35 parts by weight, still more preferably from 15 parts by weight to 27 parts by weight, with respect to 100 parts by weight of the tablet.
(2) The number of the bacteria contained in the bacterium-containing oral rapidly disintegrating tablet is from 10³ to 10²⁰, preferably from 10⁵ to 10¹⁵, more preferably from 10⁷ to 10¹³, per tablet.
(3) The blending amount of the saccharide contained in the bacterium-containing oral rapidly disintegrating tablet is not particularly limited, but is from 10 parts by weight to 90 parts by weight, preferably from 30 parts by weight to 85 parts by weight, more preferably from 50 parts by weight to 80 parts by weight, still more preferably from 70 parts by weight to 75 parts by weight, with respect to 100 parts by weight of the tablet.
(4) The tablet hardness of the bacterium-containing oral rapidly disintegrating tablet is from 15 N to 50 N, preferably from 20 N to 40 N.
(5) The moisture content of the bacterium-containing oral rapidly disintegrating tablet is 3% or less, preferably 2% or less.
(6) An oral disintegration time that is the disintegration time of the bacterium-containing oral rapidly disintegrating tablet is from 10 seconds to 30 seconds, preferably from 10 seconds to 25 seconds, more preferably from 10 seconds to 23 seconds, still more preferably from 10 seconds to 20 seconds, most preferably from 10 seconds to 19 seconds, in the mouth.
(7) The stability of viable bacteria in the bacterium-containing oral rapidly disintegrating tablet is as follows: when the amount of the viable bacteria on the day of the production of the tablet is defined as 100, the amount of the viable bacteria is from about 95 to about 100 after a lapse of 3 months, and an amount of the viable bacteria of from about 90 to about 98 (or from about 90 to about 100) can be maintained after a lapse of 6 months.

Also disclosed herein is:
A use of bacteria granulated by a spray-drying method for production of a bacterium-containing oral rapidly disintegrating tablet.

A use of bacteria granulated by a spray-drying method for a bacterium-containing oral rapidly disintegrating tablet, or a non-medical use of bacteria granulated by a spray-drying method for a bacterium-containing oral rapidly disintegrating tablet.

A use of a bacterium-containing oral rapidly disintegrating tablet, containing: bacteria; a saccharide; an excipient; a disintegrant; and a lubricant, the bacterium-containing oral rapidly disintegrating tablet being obtained by compression-forming mixture powder containing the bacteria granulated by a spray-drying method, the saccharide, the excipient, the disintegrant, and the lubricant, the bacterium-containing oral rapidly disintegrating tablet having a tablet hardness of from 15 N to 50 N.

A method for administration of a bacterium-containing oral rapidly disintegrating tablet, containing: bacteria; a saccharide; an excipient; a disintegrant; and a lubricant to a mammal (in particular, a human), the bacterium-containing oral rapidly disintegrating tablet being obtained by compression-forming mixture powder containing the bacteria granulated by a spray-drying method, the saccharide, the excipient, the disintegrant, and the lubricant, the bacterium-containing oral rapidly disintegrating tablet having a tablet hardness of from 15 N to 50 N.

The present invention is hereinafter described in detail by way of specific examples. However, the present invention is not limited to the examples.

### Example 1

### (Bacterial Powder Granulated by Spray-drying Method)

Viable bacteria (bacterial powder) granulated by a spray-drying method were prepared by the following production method.

Wet bacterial cells (viable bacterial cells) after viable bacterial culture were spray-dried in a spray-drying apparatus to provide viable bacteria in the form of fine spherical particles.

### (Bacterial Powder Granulated by Freeze-drying Method)

Viable bacteria (bacterial powder) granulated by a freeze-drying method were prepared by the following production method. The viable bacteria (bacterial powder) granulated by the freeze-drying method were used as a control.

Wet bacterial cells (viable bacterial cells) after viable bacterial culture were frozen in a chamber of a freeze-dryer, and then warmed under a reduced-pressure environment to be dried. Thus, a solid was obtained. As the freeze-dryer, Model RL-1007BF {Kyowa Vacuum Engineering Co., Ltd.} was used.

### Example 2

### (Bacterium-containing Oral Rapidly Disintegrating Tablet Using Bacterial Powder Granulated by Spray-drying Method)

A bacterium-containing oral rapidly disintegrating tablet was prepared by the following production method with the use of the bacterial powder granulated by the spray-drying method of Example 1 described above.

To the bacterial powder obtained by the spray-drying method, an appropriate excipient, disintegrant, lubricant, and the like were added, and a tablet was obtained by a compression-molding method.

### (Bacterium-containing Oral Rapidly Disintegrating Tablet Using Bacterial Powder Granulated by Freeze-drying Method)

A bacterium-containing oral rapidly disintegrating tablet was prepared by the following production method with the use of the bacterial powder granulated by the freeze-drying method of Example 1 described above. An oral rapidly disintegrating tablet containing the viable bacteria (bacterial powder) granulated by the freeze-drying method was used as a control.

To the bacterial powder obtained by the freeze-drying method, an appropriate excipient, disintegrant, lubricant, and the like were added, and a tablet was obtained by a compression-molding method.

### Example 3

### (Investigation of Characteristics of Bacterial Powder Granulated by Spray-drying Method and Bacterium-containing Oral Rapidly Disintegrating Tablet Containing the Bacterial Powder)

Characteristics of the bacterial powder granulated by the spray-drying method and the bacterium-containing oral rapidly disintegrating tablet containing the bacterial powder were investigated. The details are as described below.

### (Observation of Particles of Bacterial Powder)

Particles of bacterial powder of lactic acid bacteria granulated by the spray-drying method and bacterial powder of butyric acid bacteria granulated by the spray-drying method of Example 1, and bacterial powder of lactic acid bacteria granulated by the freeze-drying method and bacterial powder of butyric acid bacteria granulated by the freeze-drying method of Example 1 were observed with a measuring instrument (Microscope VHX-2000).

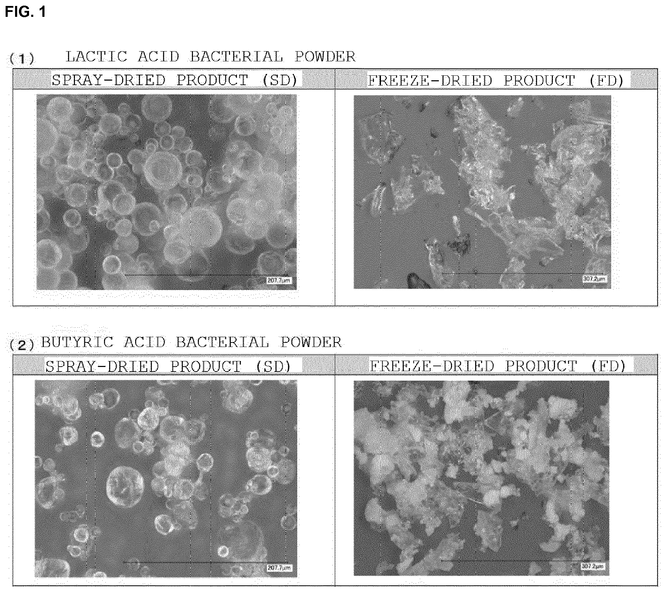

As apparent from FIG. 1(1) and FIG. 1(2), the bacterial powder of lactic acid bacteria granulated by the spray-drying method (spray-dried) and the bacterial powder of butyric acid bacteria granulated by the spray-drying method had spherical shapes and also had uniform particle diameters.

Meanwhile, as apparent from FIG. 1(1) and FIG. 1(2), the bacterial powder of lactic acid bacteria granulated by the freeze-drying method (freeze-dried) and the bacterial powder of butyric acid bacteria granulated by the freeze-drying method had amorphous particle shapes and were also not uniform in magnitude of particle diameter.

### (Measurement of Particle Diameter of Bacterial Powder)

A particle diameter was measured using a measuring instrument (laser diffraction particle diameter distribution-measuring apparatus HEROS & RODOS.).

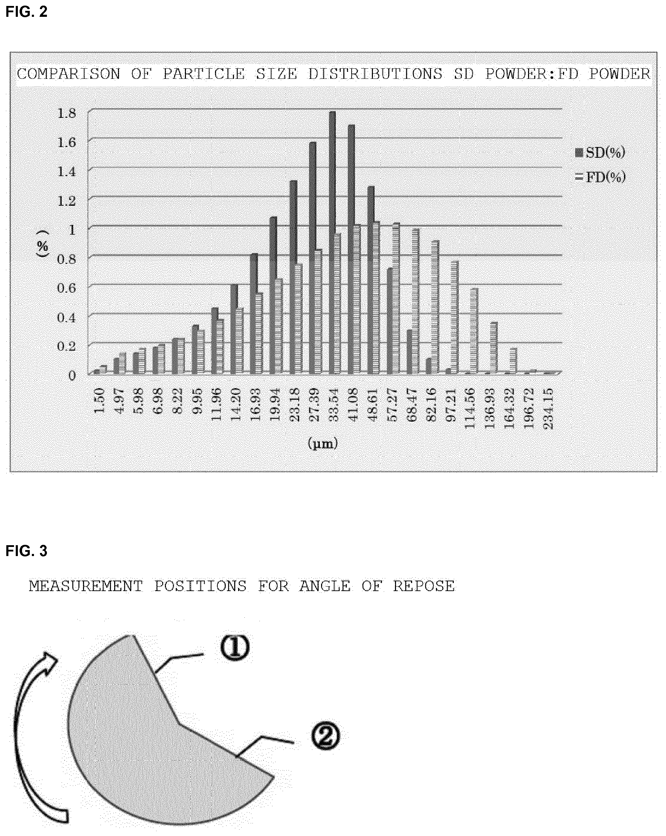

As apparent from the results of Table 1 below and FIG. 2 (bacterial powders of lactic acid bacteria), the bacterial powder granulated by the freeze-drying method has a large particle diameter distribution width, and hence requires an operation for regulating particle diameters. For example, bacterial cells having only 100-mesh sieved particle diameters can only be used.

Meanwhile, the bacterial powder granulated by the spray-drying method had a small particle diameter and also had a small particle size distribution width as compared to the bacterial powder granulated by the freeze-drying method.

**Table 1**

| | | Spray-dried product (SD) | Freeze-dried product (FD) |
|---|---|---|---|
| Particle diameter (µm) | D10 | 11.35 | 8.86 |
| | D50 | 29.28 | 39.09 |
| | D90 | 51.47 | 157.61 |

### (Investigation of Flowability)

An angle of repose serving as an indicator of the flowability of bacterial powder was measured using a measuring instrument {instrument for measuring an angle of repose based on a cylinder rotation method, Tsutsui Scientific Instruments Co., Ltd.}. Measurement angles were set to positions illustrated in FIG. 3.

As apparent from Table 2 below, the bacterial powder granulated by the spray-drying method was found to have high flowability as compared to the bacterial powder granulated by the freeze-drying method.

**Table 2**

| Bacterial powder | | | | |
|---|---|---|---|---|
| Angle of repose (°) | Using SD bacterial powder | | Using FD bacterial powder | |
| | Before | After | Before | After |
| Measurement position (1) | 57 | 45 | 60 | 51 |
| Measurement position (2) | 39 | 29 | 41 | 35 |
| *"Before" and "After" represent angles before and after collapse during right rotation. | | | | |

| Granules before tableting | | | | |
|---|---|---|---|---|
| Angle of repose (°) | Using SD bacterial powder | | Using FD bacterial powder | |
| | Before | After | Before | After |
| Measurement position (1) | 57 | 54 | 58 | 56 |
| Measurement position (2) | 39 | 32 | 40 | 34 |

| | | | | |
|---|---|---|---|---|
| *"Before" and "After" represent angles before and after collapse during right rotation. | | | | |

### (Investigation of Hardness of Tablet)

The hardnesses of the bacterium-containing oral rapidly disintegrating tablets containing lactic acid bacteria and butyric acid bacteria granulated by the spray-drying method of Example 2, and the bacterium-containing oral rapidly disintegrating tablets containing lactic acid bacteria and butyric acid bacteria granulated by the freeze-drying method of Example 2 were measured.

With regard to a measurement method, 50 tablets were randomly sampled, and the hardness of each of the tablets was measured using a measuring instrument (Hardness Meter Model KHT-20N).

As shown in the measurement results of Table 3 below, the bacterium-containing oral rapidly disintegrating tablets containing the bacterial powders granulated by the spray-drying method were able to be easily caused to approach the target tablet hardness range.

**Table 3**

| Unit: (N), Hardness target range (mean): 28±3 N | | | |
|---|---|---|---|
| SD | | FD | |
| Mean | Range | Mean | Range |
| 26.4 | 20.5-30.0 | 28.5 | 21.0-33.0 |

### (Investigation of Disintegration Time of Tablet)

The disintegration times of the bacterium-containing oral rapidly disintegrating tablets containing lactic acid bacteria and butyric acid bacteria granulated by the spray-drying method of Example 2, and the bacterium-containing oral rapidly disintegrating tablets containing lactic acid bacteria and butyric acid bacteria granulated by the freeze-drying method of Example 2 were measured.

A measurement method for the disintegration time is as described below.
Measurement method: a basket method using a beaker based on a disintegration test method in the Japanese Pharmacopoeia
Measuring instrument: Disintegration Tester Model NT-200 {manufactured by Toyama Sangyo Co., Ltd.}
Test liquid: purified water (without a disc)
Test results: shown as "shortest time"-"longest time" of six tablets in Table 4 below.

As shown in the measurement results of Table 4 below, the bacterium-containing oral rapidly disintegrating tablets containing the bacterial powders granulated by the spray-drying method were found to be able to shorten the disintegration time as compared to the bacterium-containing oral rapidly disintegrating tablets containing the bacterial powders granulated by the freeze-drying method.

**Table 4**

| Unit: seconds | | |
|---|---|---|
| | SD | FD |
| | 17-18 | 20-21 |

### (Investigation of Weight Variation)

The weight variations of the bacterium-containing oral rapidly disintegrating tablet using the bacterial powder granulated by the spray-drying method of Example 2 and the bacterium-containing oral rapidly disintegrating tablet using the bacterial powder granulated by the freeze-drying method of Example 2 were measured.

As shown in the measurement results of Table 5 below, the bacterium-containing oral rapidly disintegrating tablet using the bacterial powder granulated by the spray-drying method was found to have a small weight variation as compared to the bacterium-containing oral rapidly disintegrating tablet using the bacterial powder granulated by the freeze-drying method. That is, the bacterium-containing oral rapidly disintegrating tablet containing the bacterial powder granulated by the spray-drying method was found to have a low tablet-to-tablet variation in content of bacteria.

**Table 5**

| | Tablet using SD bacterial powder | | | Tablet using FD bacterial powder | | |
|---|---|---|---|---|---|---|
| | (1) | (2) | (3) | (1) | (2) | (3) |
| Mean | 101.1 | 101.0 | 101.1 | 100.4 | 100.5 | 100.5 |
| Min | 100.3 | 99.9 | 100.1 | 99.0 | 99.4 | 98.9 |
| Max | 102.0 | 101.8 | 101.8 | 101.6 | 101.6 | 101.4 |
| Weight variation | 0.42 | 0.43 | 0.44 | 0.54 | 0.47 | 0.50 |

| | | | | | | |
|---|---|---|---|---|---|---|
| Unit: mg | | | | | | |

### (Investigation of Viable Bacterial Count Uniformity)

The viable bacterial count uniformity of each of the bacterial powders containing lactic acid bacteria and butyric acid bacteria granulated by the spray-drying method of Example 1, the bacterial powders containing lactic acid bacteria and butyric acid bacteria granulated by the freeze-drying method of Example 1, the bacterium-containing oral rapidly disintegrating tablets containing the bacterial powders containing lactic acid bacteria and butyric acid bacteria granulated by the spray-drying method of Example 2, and the bacterium-containing oral rapidly disintegrating tablets containing the bacterial powders containing lactic acid bacteria and butyric acid bacteria granulated by the freeze-drying method of Example 2 was investigated.
Measurement conditions are as described below.
Viable bacterial count test method (quantitative, in conformity to the Japanese Pharmaceutical Codex)

An undiluted sample solution was diluted to a concentration of containing 20 to 200 viable bacteria in 1 mL thereof, and was solidified by adding a testing medium. Culture was performed in a predetermined temperature range for a specified period of time, and a bacterial count was determined from the number of colonies that had appeared.

The measurement results of the viable bacterial count uniformity for the bacterial powders and the oral rapidly disintegrating tablets containing the bacterial powders are shown in Tables 6 and 7 below. As shown in the measurement results of Tables 6 and 7 below, the bacterial powders containing lactic acid bacteria and butyric acid bacteria granulated by the spray-drying method and the bacterium-containing oral rapidly disintegrating tablets containing the bacterial powders were found to have high viable bacterial count uniformity as compared to the bacterial powders containing lactic acid bacteria and butyric acid bacteria granulated by the freeze-drying method and the bacterium-containing oral rapidly disintegrating tablets containing the bacterial powders, respectively.

**Table 6**

| Viable bacterial count uniformity of mixture powder | | | | |
|---|---|---|---|---|
| cfu/g | | | | |
| | Lactic acid bacteria | | Butyric acid bacteria | |
| | SD | FD | SD | FD |
| (1) | 4.3E+08 | 5.1E+08 | 1.8E+08 | 1.4E+08 |
| (2) | 4.4E+08 | 4.3E+08 | 1.7E+08 | 1.9E+08 |
| (3) | 4.2E+08 | 4.0E+08 | 1.8E+08 | 1.5E+08 |
| (4) | 4.8E+08 | 6.4E+08 | 1.9E+08 | 1.8E+08 |
| (5) | 4.0E+08 | 4.6E+08 | 1.8E+08 | 1.5E+08 |
| (6) | 4.0E+08 | 6.8E+08 | 1.8E+08 | 1.9E+08 |
| (7) | 4.7E+08 | 4.5E+08 | 1.8E+08 | 1.4E+08 |
| (8) | 4.4E+08 | 6.5E+08 | 1.7E+08 | 1.4E+08 |
| Mean | 4.4E+08 | 5.3E+08 | 1.8E+08 | 1.6E+08 |
| Standard deviation σ | 2.9E+07 | 1.1E+08 | 6.4E+06 | 2.3E+07 |
| RSD (%) | 6.73 | 21.20 | 3.59 | 14.17 |

**Table 7**

| Viable bacterial count uniformity of tablet | | | | |
|---|---|---|---|---|
| cfu/tablet | | | | |
| | Lactic acid bacteria | | Butyric acid bacteria | |
| | SD \| | FD | SD | FD |
| 1 | 5.1E+07 | 3.6E+07 | 8.1E+06 | 1.2E+07 |
| 2 | 5.2E+07 | 3.9E+07 | 1.0E+07 | 1.1E+07 |
| 3 | 5.9E+07 | 3.3E+07 | 8.2E+06 | 1.1E+07 |
| 4 | 5.3E+07 | 3.6E+07 | 7.6E+06 | 9.6E+06 |
| 5 | 4.5E+07 | 3.4E+07 | 7.8E+06 | 9.2E+06 |
| 6 | 4.9E+07 | 3.2E+07 | 1.0E+07 | 8.4E+06 |
| 7 | 5.3E+07 | 3.0E+07 | 7.8E+06 | 8.2E+06 |
| 8 | 5.5E+07 | 2.8E+07 | 8.8E+06 | 1.1E+07 |
| Mean | 5.2E+07 | 3.3E+07 | 8.6E+06 | 1.0E+07 |
| σ | 4.1E+06 | 3.5E+06 | 1.0E+06 | 1.4E+06 |
| RSD (%) | 7.8 | 10.6 | 11.9 | 13.9 |

### (Investigation of Stability of Bacterial Powder)

The stability (viability) of viable bacteria was investigated using the bacterial powder of lactic acid bacteria and bacterial powder of butyric acid bacteria granulated by the spray-drying method of Example 1, and the bacterial powder of lactic acid bacteria and bacterial powder of butyric acid bacteria granulated by the freeze-drying method of Example 1.
Conditions for an acceleration test are as described below.
Stability test (acceleration test)
Storage conditions: 40°C±2°C/75%RH±5%RH
Test period: 6 months

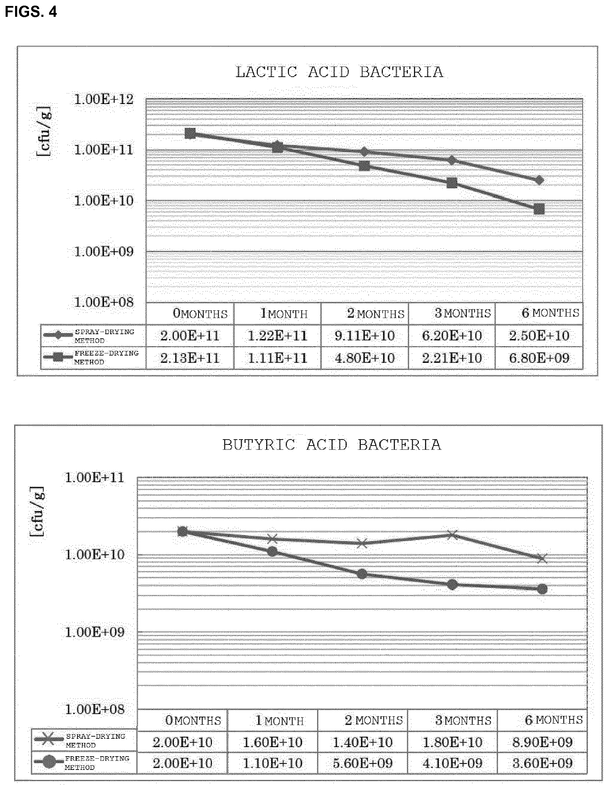

As apparent from the results of FIG. 4, the stability (viability) of the bacterial powders granulated by the spray-drying method was found to be at least 2.5-fold higher at a lapse of 6 months from production as compared to the stability (viability) of the bacterial powders granulated by the freeze-drying method.

The results support the following: the bacterium-containing oral rapidly disintegrating tablet containing the bacterial powder granulated by the spray-drying method has high stability (viability) of viable bacteria in the tablet as compared to the bacterium-containing oral rapidly disintegrating tablet containing the bacterial powder granulated by the freeze-drying method.

### (Investigation of Stability of Bacterium-containing Oral Rapidly Disintegrating Tablet)

The stability (viability) of viable bacteria was investigated using the bacterium-containing oral rapidly disintegrating tablet containing lactic acid bacteria or butyric acid bacteria granulated by the spray-drying method (SD) of Example 2, and the bacterium-containing oral rapidly disintegrating tablet containing lactic acid bacteria or butyric acid bacteria granulated by the freeze-drying method (FD) of Example 2.

Conditions for an acceleration test are as described below.
Stability test (acceleration test)
Storage conditions: 40°C±2°C/75%RH±5%RH
Test period: 3 months

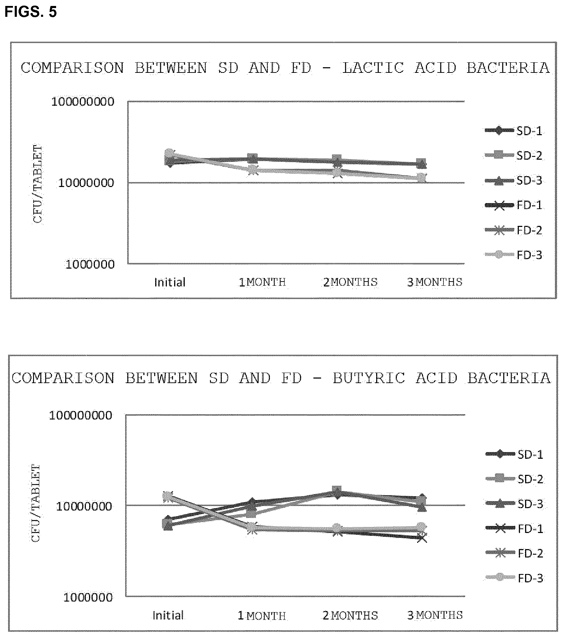

As apparent from the results of Table 8 below and FIG. 5, the results of the stability (viability) of the bacterial powders granulated by the spray-drying method were higher in the bacterium-containing oral rapidly disintegrating tablets using the bacterial powders granulated by the spray-drying method for both of the lactic acid bacteria and the butyric acid bacteria as compared to the stability (viability) of the bacterial powder granulated by the freeze-drying method. Specifically, the viability was about 1.5-fold higher for the lactic acid bacteria, and was about 2.2-fold higher for the butyric acid bacteria.

That is, the bacterium-containing oral rapidly disintegrating tablet containing the bacterial powder granulated by the spray-drying method was found to have high stability (viability) of viable bacteria in the tablet as compared to the bacterium-containing oral rapidly disintegrating tablet containing the bacterial powder granulated by the freeze-drying method.

In general, a bacterium-containing oral rapidly disintegrating tablet has additives, such as an excipient, a disintegrant, and a lubricant, blended in its formulation in addition to bacterial powder. Further, in triturating, mixing, tableting, and packaging steps in a tablet production process, a reduction in activity of viable bacteria due to physical breakage, moisture absorption, or the like occurs. However, the bacterium-containing oral rapidly disintegrating tablet containing the bacterial powder granulated by the spray-drying method of the present invention was found to maintain excellent stability of viable bacteria even after undergoing those steps.

**Table 8**

| [Lactic acid bacteria] | | | | |
|---|---|---|---|---|
| cfu/tablet | | | | |
| Lot. No. | Initial | 1 month | 2 months | 3 months |
| SD-1 | 1.7E+07 | 1.9E+07 | 1.8E+07 | 1.7E+07 |
| SD-2 | 1.8E+07 | 1.9E+07 | 1.9E+07 | 1.7E+07 |
| SD-3 | 1.9E+07 | 1.9E+07 | 1.8E+07 | 1.7E+07 |
| FD-1 | 2.2E+07 | 1.4E+07 | 1.3E+07 | 1.1E+07 |
| FD-2 | 2.2E+07 | 1.4E+07 | 1.4E+07 | 1.1E+07 |
| FD-3 | 2.3E+07 | 1.4E+07 | 1.3E+07 | 1.1E+07 |

| [Butyric acid bacteria] | | | | |
|---|---|---|---|---|
| cfu/tablet | | | | |
| Lot. No. | Initial | 1 month | 2 months | 3 months |
| SD-1 | 7.0E+06 | 1.1E+07 | 1.3E+07 | 1.2E+07 |
| SD-2 | 6.2E+06 | 8.0E+06 | 1.4E+07 | 1.1E+07 |
| SD-3 | 6.1E+06 | 9.7E+06 | 1.4E+07 | 9.6E+06 |
| FD-1 | 1.3E+07 | 5.9E+06 | 5.1E+06 | 4.4E+06 |
| FD-2 | 1.2E+07 | 5.4E+06 | 5.2E+06 | 5.2E+06 |
| FD-3 | 1.2E+07 | 5.7E+06 | 5.5E+06 | 5.7E+06 |

### Example 4

### (Production of Bacterium-containing Oral Rapidly Disintegrating Tablet of the Present Invention)

The bacterium-containing oral rapidly disintegrating tablet of the present invention was produced so as to have a compositional ratio shown in Table 8 below.

Lactic acid bacteria {*Streptococcus faecalis* T-110} granulated by the spray-drying method, saccharifying bacteria *{Bacillus mesentericus* TO-A} granulated by the spray-drying method, butyric acid bacteria *{Clostridium butyricum* TO-A} granulated by the spray-drying method, corn starch (as necessary), lactose hydrate, microcrystalline cellulose (as necessary), anhydrous dibasic calcium phosphate, crospovidone (as necessary), and light anhydrous silicic acid (as necessary) were added, and lubricants {magnesium stearate and sodium stearyl fumarate (as necessary)} were further added, followed by mixing using a rotary rocking mixer for 5 minutes. The resultant mixture powder was stored in a polyethylene container until the next step.

Next, the mixture powder was tableted using a rotary tableting machine so as to achieve a tablet mass target (mean) of 100±5 mg and a tablet hardness target (mean) of 28±3 N. Thus, a bacterium-containing oral rapidly disintegrating tablet was produced.

**Table 9**

| Purpose of blending | Standard | Component name | Composition Example 1 | Composition Example 2 | Composition Example 3 |
|---|---|---|---|---|---|
| Active component | Japanese Pharmaceutical Codex | Lactic acid bacteria | 12.0 | 12.0 | 12.0 |
| | Japanese Pharmaceutical Codex | Saccharifying bacteria | 60.0 | 60.0 | 60.0 |
| | Standard in appendix | Butyric acid bacteria | 60.0 | 60.0 | 60.0 |
| Excipient | Japanese Pharmacopoeia | Corn starch | 50.0 | | 50.0 |
| Excipient | Japanese Pharmacopoeia | Potato starch | | 50.0 | |
| Excipient | Japanese Pharmacopoeia | Lactose hydrate | 305.0 | 313.5 | 330.5 |
| Excipient | Japanese Pharmacopoeia | Mannitol | | | |
| Excipient | Japanese Pharmacopoeia | Low-substituted hydroxypropylcellulose | 40.0 | 30.0 | |
| Excipient | Japanese Pharmacopoeia | Microcrystalline cellulose | | | 30.0 |
| Excipient | Japanese Pharmacopoeia | Anhydrous dibasic calcium phosphate | 50.0 | 40.0 | 30.0 |
| Disintegrant | Japanese Pharmaceutical Excipients | Crospovidone | 20.0 | | 25.0 |
| Disintegrant | Japanese Pharmacopoeia | Carmellose | | 30.0 | |
| Disintegration aid | Japanese Pharmacopoeia | Light anhydrous silicic acid | | | 2.0 |
| Lubricant | Japanese Pharmacopoeia | Talc | | 2.0 | |
| Lubricant | Japanese Pharmacopoeia | Magnesium stearate | | 0.5 | 0.5 |
| Lubricant | Japanese Pharmaceutical Excipients | Sodium stearyl fumarate | 3.0 | 2.0 | |
| | | Total | 600.0 | 600.0 | \| 600.0 I |
| | | | | | |
| | | Evaluation criteria | Composition Example 1 | Composition Example 2 | Composition Example 3 |
| | | Hardness (N) | 25 to 40 (⊚) | 25 to 35 | 20 to 40 (⊚) |
| | | Loss on drying (%) | 2 to 3 (○) | 2 to 3 (○) | 2 to 3 (○) |
| | | Disintegration (seconds) time | 18 to 28 (○) | 18 to 30 (○) | 18 to 24 (⊚) |

Characteristics of the bacterium-containing oral rapidly disintegrating tablets in this Example are as described below.
Tablet hardness: 20 N to 40 N
Moisture content: 2% to 3% or less
Disintegration time: 18 seconds to 30 seconds
Stability (viability) of viable bacteria similar to that in Example 3

### Example 5

### (Characteristics of Bacterium-containing Oral Rapidly Disintegrating Tablet of the Present Invention)

An example of the bacterium-containing oral rapidly disintegrating tablet of the present invention is as described below.
Active component: 2 mg of lactic acid bacteria, 10 mg of butyric acid bacteria, and 10 mg of saccharifying bacteria, per tablet
Additive: Talc, sodium stearyl fumarate, anhydrous dibasic calcium phosphate, potato starch, lactose hydrate, low-substituted hydroxypropylcellulose, crospovidone, and light anhydrous silicic acid
Dosage form: Plain tablet (orally disintegrating tablet), diameter: 6.5 mm, thickness: 2.5 mm, weight: about 100 mg
Efficacy or effect: Amelioration of various symptoms due to abnormalities in intestinal flora (in particular, gastroenteritis, diarrhea, dyspeptic diarrhea, constipation, acute/chronic enteritis, alternating diarrhea and constipation, and irritable bowel syndrome)
Usage and dosage: Three to six tablets are generally orally administered a day for an adult in three divided doses. The dosage is increased or reduced as appropriate depending on age and symptoms.

### (General Remark)

As apparent from Examples described above, the bacterium-containing oral rapidly disintegrating tablet of the present invention has the following remarkable effects as compared to the related-art bacterium-containing oral rapidly disintegrating tablet.

The bacterial powder granulated by the spray-drying method of the present invention has the following effects as compared to the bacterial powder granulated by the freeze-drying method.
(1) The particle shapes are spherical and uniform.
(2) The particle diameters are uniform.
(3) The flowability is high.

Further, the bacterium-containing oral rapidly disintegrating tablet containing the bacterial powder granulated by the spray-drying method of the present invention has the following effects as compared to the bacterium-containing oral rapidly disintegrating tablet containing the bacterial powder granulated by the freeze-drying method.
(1) The disintegration time is short.
(2) The viable bacterial count uniformity among tablets is high.
(3) The stability (viability) of viable bacteria is high.

### Industrial Applicability

According to the present invention, the bacterium-containing oral rapidly disintegrating tablet having excellent oral rapid disintegrability and excellent stability of viable bacteria can be provided.

## Claims

1. A bacterium-containing oral rapidly disintegrating tablet, comprising:
bacteria comprising viable bacteria;
a saccharide;
an excipient;
a disintegrant; and
a lubricant,
the bacterium-containing oral rapidly disintegrating tablet being obtained by compression-forming mixture powder containing the bacteria comprising viable bacteria granulated by a spray-drying method, the saccharide, the excipient, the disintegrant, and the lubricant,
the bacterium-containing oral rapidly disintegrating tablet having a tablet hardness of from 15 N to 50 N.

2. A bacterium-containing oral rapidly disintegrating tablet according to claim 1, wherein the bacterium-containing oral rapidly disintegrating tablet has a moisture content of 3% or less.

3. A bacterium-containing oral rapidly disintegrating tablet according to any one of claims 1 or 2, wherein the bacteria include at least bacteria of one or more members selected from lactic acid bacteria, butyric acid bacteria, and hay bacilli.

4. A bacterium-containing oral rapidly disintegrating tablet according to claim 3, wherein the hay bacilli comprise saccharifying bacteria.

5. A bacterium-containing oral rapidly disintegrating tablet according to claim 4, wherein the lactic acid bacteria comprise *Streptococcus faecalis,* the butyric acid bacteria comprise *Clostridium butyricum,* and the saccharifying bacteria comprise *Bacillus mesentericus.*

6. A bacterium-containing oral rapidly disintegrating tablet according to claim 5, wherein the lactic acid bacteria comprise *Streptococcus faecalis* T-110 *(Enterococcus faecium* T-110), the butyric acid bacteria comprise *Clostridium butyricum* TO-A, and the saccharifying bacteria comprise *Bacillus mesentericus* TO-A *(Bacillus subtilis* TO-A).

7. A bacterium-containing oral rapidly disintegrating tablet according to any one of claims 1 to 6, wherein the bacterium-containing oral rapidly disintegrating tablet has a mass of from 85 mg to 115 mg.

8. A production method for a bacterium-containing oral rapidly disintegrating tablet, comprising the steps of:
(1) granulating bacteria that comprise viable bacteria by a spray-drying method;
(2) mixing the bacteria granulated in the step (1), a saccharide, an excipient, a disintegrant, and a lubricant to produce mixture powder; and
(3) compression-forming the mixture powder of the step (2) so as to achieve a tablet hardness of from 15 N to 50 N.

9. A production method according to claim 8, wherein a tableting pressure in the compression-forming is from 5 kN to 10 kN.

## Patentansprüche

1. Bakterienhaltige, oral schnell zerfallende Tablette, umfassend:
Bakterien, die lebensfähige Bakterien umfassen;
ein Saccharid;
einen Hilfsstoff;
ein Sprengmittel; und
ein Gleitmittel,
wobei die bakterienhaltige, oral schnell zerfallende Tablette durch Verpressformen einer Pulvermischung erhalten wird, das die Bakterien, die lebensfähige Bakterien umfassen und die durch ein Sprühtrocknungsverfahren granuliert wurden, das Saccharid, den Hilfsstoff, das Sprengmittel und das Gleitmittel enthält,
wobei die bakterienhaltige, oral schnell zerfallende Tablette eine Tablettenhärte von 15 N bis 50 N aufweist.

2. Bakterienhaltige, oral schnell zerfallende Tablette nach Anspruch 1, wobei die bakterienhaltige, oral schnell zerfallende Tablette einen Feuchtigkeitsgehalt von 3 % oder weniger aufweist.

3. Bakterienhaltige, oral schnell zerfallende Tablette nach einem der Ansprüche 1 oder 2, wobei die Bakterien mindestens Bakterien eines oder mehrerer Mitglieder umfassen, die aus Milchsäurebakterien, Buttersäurebakterien und Heubakterien ausgewählt sind.

4. Bakterienhaltige, orale schnell zerfallende Tablette nach Anspruch 3, wobei die Heubakterien verzuckernde Bakterien umfassen.

5. Bakterienhaltige, oral schnell zerfallende Tablette nach Anspruch 4, wobei die Milchsäurebakterien *Streptococcus faecalis,* die Buttersäurebakterien *Clostridium butyricum* und die verzuckernden Bakterien *Bacillus mesentericus* umfassen.

6. Bakterienhaltige, oral schnell zerfallende Tablette nach Anspruch 5, wobei die Milchsäurebakterien *Streptococcus faecalis* T-110 *(Enterococcus faecium* T-110) umfassen, die Buttersäurebakterien *Clostridium butyricum* (TO-A) umfassen und die verzuckernden Bakterien *Bacillus mesentericus* TO-A *(Bacillus subtilis* TO-A) umfassen.

7. Bakterienhaltige, oral schnell zerfallende Tablette nach einem der Ansprüche 1 bis 6, wobei die bakterienhaltige, oral schnell zerfallende Tablette eine Masse von 85 mg bis 115 mg aufweist.

8. Herstellungsverfahren für eine bakterienhaltige, oral schnell zerfallende Tablette, umfassend die Schritte des :
(1) Granulierens von Bakterien, die lebensfähige Bakterien umfassen, durch ein Sprühtrocknungsverfahren;
(2) Mischen der in Schritt (1) granulierten Bakterien, eines Saccharids, eines Hilfsstoffs, eines Sprengmittels und eines Gleitmittels, um eine Pulvermischung herzustellen; und
(3) Verpressformen der Pulvermischung aus Schritt (2), um eine Tablettenhärte von 15 N bis 50 N zu erzielen.

9. Herstellungsverfahren nach Anspruch 8, wobei der Tablettierdruck beim Verpressformen 5 kN bis 10 kN beträgt.

## Revendications

1. Comprimé oral à désintégration rapide contenant une bactérie, comprenant :
des bactéries comprenant des bactéries viables ;
un saccharide ;
un excipient ;
un désintégrant ; et
un lubrifiant,
le comprimé oral à désintégration rapide contenant une bactérie étant obtenu par compression-formation d'un mélange de poudre contenant les bactéries comprenant des bactéries viables granulées par un procédé de séchage par pulvérisation, le saccharide, l'excipient, le désintégrant et le lubrifiant,
le comprimé oral à désintégration rapide contenant une bactérie ayant une dureté de comprimé de 15 N à 50 N.

2. Comprimé oral à désintégration rapide contenant une bactérie selon la revendication 1, dans lequel le comprimé oral à désintégration rapide contenant une bactérie a une teneur en humidité de 3 % ou moins.

3. Comprimé oral à désintégration rapide contenant une bactérie selon l'une quelconque des revendications 1 ou 2, dans lequel les bactéries comportent au moins des bactéries d'un ou plusieurs éléments choisis parmi les bactéries d'acide lactique, les bactéries d'acide butyrique et les bacilles du foin.

4. Comprimé oral à désintégration rapide contenant une bactérie selon la revendication 3, dans lequel les bacilles du foin comprennent des bactéries saccharifiantes.

5. Comprimé oral à désintégration rapide contenant une bactérie selon la revendication 4, dans lequel les bactéries d'acide lactique comprennent *Streptococcus faecalis,* les bactéries d'acide butyrique comprennent *Clostridium butyricum* et les bactéries saccharifiantes comprennent *Bacillus mesentericus.*

6. Comprimé oral à désintégration rapide contenant une bactérie selon la revendication 5, dans lequel les bactéries d'acide lactique comprennent *Streptococcus faecalis* T-110 *(Enterococcus faecium* T-110), les bactéries d'acide butyrique comprennent *Clostridium butyricum* TO-A, et les bactéries saccharifiantes comprennent *Bacillus mesentericus* TO-A *(Bacillus subtilis* TO-A).

7. Comprimé oral à désintégration rapide contenant une bactérie selon l'une quelconque des revendications 1 à 6, dans lequel le comprimé oral à désintégration rapide contenant une bactérie a une masse de 85 mg à 115 mg.

8. Procédé de production d'un comprimé oral à désintégration rapide contenant une bactérie, comprenant les étapes :
(1) de granulation de bactéries qui comprennent des bactéries viables par un procédé de séchage par pulvérisation ;
(2) de mélange des bactéries granulées à l'étape (1), un saccharide, un excipient, un désintégrant et un lubrifiant pour produire un mélange de poudre ; et
(3) de compression-formation du mélange de poudre de l'étape (2) de façon à obtenir une dureté de comprimé de 15 N à 50 N.

9. Procédé de production selon la revendication 8, dans lequel une pression de compression dans la compression-formation est de 5 kN à 10 kN.
